# EUROPÄISCHE PATENTANMELDUNG

(11) **EP 2 977 837 A1**
(43) Veröffentlichungstag der Anmeldung: **27.01.2016**
(21) Anmeldenummer: 15177888.3
(22) Anmeldetag: 22.07.2015
(51) Int. Cl.: G05B 13/04, G05B 23/02, G05B 17/02, C10G 9/00

(54) **VERFAHREN UND SYSTEM ZUR AUSWERTUNG EINES CHEMISCHEN VERARBEITUNGSPROZESSES**

(30) Priorität: 22.07.2014 AT 505072014
(71) Anmelder: OMV REFINING & MARKETING GMBH, 1020 Wien (AT)
(72) Erfinder: Bacher, Wolfgang, 2440 Gramatneusiedl (AT)
(74) Vertreter: Sonn & Partner Patentanwälte

(57) **Zusammenfassung**

Computergestütztes Verfahren und System zur Analyse eines chemischen Verarbeitungsprozesses, bei welchem aus einem Ausgangsstoff (1) ein Produkt (3) erzeugt wird, dessen Produktzusammensetzung neben einer Ausgangszusammensetzung des Ausgangsstoffs (1) von zumindest zwei Betriebsparametern des Verarbeitungsprozesses abhängt, durch Simulation des Verarbeitungsprozesses, wobei
- die Ausgangszusammensetzung gemessen oder vorgegeben wird,
- ausgehend von der Ausgangszusammensetzung und unter Variation der zumindest zwei Betriebsparameter eine Reaktionsfläche (14) von erwarteten Produktzusammensetzungen simuliert wird, wobei jeder Punkt auf der Reaktionsfläche (14) zumindest einer Konfiguration der Betriebsparameter entspricht,
- eine Produktzusammensetzung (25; 26; 27; 32, 33) zumindest teilweise gemessen wird, und
- die zumindest teilweise gemessenen Produktzusammensetzung (25; 26; 27; 32; 33) mit der Reaktionsfläche (14) zur Erkennung von Mess- oder Simulationsfehlern oder zur Ergänzung der gemessenen Produktzusammensetzung (26) in Beziehung gesetzt wird.

## Beschreibung

Die Erfindung betrifft ein computergestütztes Verfahren und ein System zur Analyse eines chemischen Verarbeitungsprozesses, bei welchem aus einem Ausgangsstoff ein Produkt erzeugt wird, dessen Produktzusammensetzung neben einer Ausgangszusammensetzung des Ausgangsstoffs von zumindest zwei Betriebsparametern des Verarbeitungsprozesses abhängt, durch Simulation des Verarbeitungsprozesses.

Insbesondere betrifft die Erfindung ein Verfahren zur Optimierung eines chemischen Verarbeitungsprozesses. Damit ist jegliche Verbesserung des Prozesses, d.h. jede Veränderung des Prozesses, welche zu einer höheren Ausbeute an Produkten bzw. zu einem höheren Gesamtwert der aus dem Prozess erhaltenen Produkte führt, gemeint. Darunter fallen insbesondere die Überwachung der den Prozess beeinflussenden Messgrößen und Analysen auf Fehler, die Bestimmung von Fehlern und Korrektur eines der Prozesssteuerung zugrunde liegenden Prozessmodells, die Kalibrierung einer aus Messungen abgeleiteten Vorhersage hinsichtlich der erzielbaren Ausbeute, welche zur Planung und Steuerung des Prozesses verwendet wird, sowie die Ergänzung und Extrapolation von bei einer Probennahme und Aufbereitung verlorenen oder verfälschten Produktkomponenten zur Vervollständigung der Prozessüberwachung und -steuerung. Mit einem chemischen Verarbeitungsprozess ist hier grundsätzlich jedes Verfahren gemeint, welches durch eine zumindest teilweise Stoffwandlung mit einer oder mehreren chemischen Reaktionen einen Ausgangsstoff in ein Produkt verarbeitet. Insbesondere sind davon petrochemische Verarbeitungsprozesse umfasst, z.B. ein Dampfspaltungsprozess, bei dem aus einem Einsatz oder einer Mischung aus mehreren Einsätzen (Ausgangsstoff) ein Spaltgas (Produkt) hergestellt wird. Grundsätzlich ist die Anwendbarkeit der Erfindung aber nicht auf Raffinerien oder dergleichen beschränkt, sondern schließt jeden chemischen Verarbeitungsprozess ein, bei dem eine zu erwartende Produktzusammensetzung anhand einer bekannten oder angenommenen Ausgangszusammensetzung und zumindest zwei Betriebsparametern abgeschätzt, berechnet oder simuliert werden kann. Dementsprechend umfasst der Begriff Verarbeitungsprozess auch im Labor, z.B. während einer Spaltgasanalyse, durchgeführte Prozesse, bei denen ein Ausgangsstoff mit einer Ausgangszusammensetzung in eine davon verschiedene Produktzusammensetzung übergeführt wird. Als Betriebsparameter werden hierbei sämtliche variablen Prozessparameter abgesehen von der Zusammensetzung des Ausgangsstoffs bzw. des Produkts, insbesondere sämtliche Steuergrößen des Verarbeitungsprozesses, bezeichnet. Der Ausgangsstoff und das Produkt sind dabei jeweils im Allgemeinen ein Gemisch aus einer oder mehreren chemischen Komponenten, d.h. chemischen Elementen oder Verbindungen. Die Ausgangszusammensetzung, d.h. die Zusammensetzung des Ausgangsstoffs, oder die Produktzusammensetzung, d.h. die Zusammensetzung des Produkts, kann üblicherweise durch die Anteile (z.B. Volumenprozent oder Gewichtsprozent) einer oder mehrerer Komponenten am Ausgangsstoff bzw. am Produkt angegeben werden. Aufgrund der dem Prozess eigenen Stoffwandlung unterscheidet sich die Produktzusammensetzung im Allgemeinen von der Ausgangszusammensetzung. Für die Zwecke der Erfindung, welche sich insbesondere für den Einsatz im Zusammenhang mit Gleichgewichtsreaktionen eignet, kommt es nicht auf die Richtung des Ablaufs oder die sonstigen Eigenschaften einer bestimmten Reaktion an, d.h. im Folgenden sind die Begriffe Ausgangsstoff und Produkt für das Verständnis und die Anwendbarkeit der Erfindung praktisch austauschbar. Vorzugsweise handelt es sich bei dem gegenständlichen Verfahren um ein computergestütztes Verfahren. Die Simulation des Verarbeitungsprozess und/oder die Assoziation zwischen der gemessenen Produktzusammensetzung und der Reaktionsfläche wird dementsprechend vorzugsweise auf einem dafür eingerichteten Computer durchgeführt bzw. ermittelt.

Derartige Verarbeitungsprozesse werden typischerweise detailliert überwacht, wobei insbesondere die Ausgangszusammensetzung und die Produktzusammensetzung gemessen werden. Anhand der gemessenen Zusammensetzungen können der ordnungsgemäße Ablauf des Prozesses sichergestellt und vorangehende und/oder nachfolgende Verarbeitungsschritte, insbesondere weitere, unabhängig ablaufende Verarbeitungsprozesse (z.B. die Aufarbeitung der aus dem Verarbeitungsprozess erhaltenen Produkte), entsprechend geplant und gesteuert werden. Falls die Überwachung ausreichend aktuell ist bzw. annähernd in Echtzeit erfolgt, kann anhand der gemessenen oder ermittelten Werte eine vorteilhafte Regelung des Verarbeitungsprozesses vorgenommen werden. Insbesondere bei modellbasierten Anwendungen, wie einer Modellprädiktive Regelung ("Model Predictive Control"; MPC), wird anhand der gemessenen Größen eine optimale Zielvorgabe für den Prozess ermittelt. Bei der Überwachung treten jedoch naturgemäß Messfehler auf, welche die Planung, Steuerung oder Regelung, sowie etwaige aus den Messungen abgeleitete Zielvorgaben, verfälschen und somit den Prozess negativ beeinflussen und von einem optimalen Verlauf sowie einer optimalen Ausbeute wegführen. Solche Messfehler können üblicherweise daran erkannt werden, dass die ermittelten Messwerte inkonsistent sind bzw. sich scheinbar widersprechen. Aufgrund der Komplexität der Zusammenhänge zwischen den einzelnen Messgrößen ist es jedoch in vielen Fällen schwierig, solche Messfehler zuzuordnen, geschweige denn in adäquater Weise zu korrigieren.

Die EP 1 112 532 B1 beschreibt ein System und Verfahren zur Feststellung von Sensorfehlern oder -ausfällen, welches auf beliebige Prozesse anwendbar ist, weil es auf allgemeinen statistischen Methoden basiert und nicht auf Ausgangszusammensetzungen und Produktzusammensetzungen eines chemischen Verarbeitungsprozesses beschränkt ist. Dementsprechend kann das gezeigte Verfahren die dem chemischen Verarbeitungsprozess inhärenten Gesetzmäßigkeiten nicht ausnutzen. Stattdessen wird ein lineares Modell der erwarteten Korrelationen zwischen den Messwerten zur Feststellung eines Fehlers, Identifikation der fehlerhaften Messwerts und Abschätzung eines korrigierten Messwerts verwendet. Der Einfluss von Prozessparametern, insbesondere gesteuerten Betriebsparametern, auf den überwachten Prozess und somit auch auf die Korrelationen zwischen den Messwerten wird dabei jedoch nicht berücksichtigt. In das dem Verfahren zugrunde liegende lineare Modell gehen lediglich die Messwerte selbst als Freiheitsgrade ein. Die in Abhängigkeit von den Betriebsparametern unterschiedlichen, Arbeitspunkt-spezifischen Korrelationen können daher bei dem in EP 1 112 532 B1 gezeigten Verfahren nicht zur Erkennung und Korrektur von Sensorfehlern genutzt werden.

Das aus WO 1994/028557 A1 bekannte Verfahren betrifft ebenfalls beliebige Prozessvariablen; konkret offenbart ist eine Anwendung in einem Brutreaktor (EBR-II) und die Überwachung des Kühlsystems sowie des Neutronenflusses dieses Reaktors. D.h. auch dieses Verfahren kann nicht auf den Gesetzmäßigkeiten chemischer Reaktionen beruhen. Dasselbe gilt auch für das aus EP 1 643 332 A2 (D3) bekannte Verfahren, welches eine Anwendung bei Motoren von Gasturbinen betrifft.

Es ist Aufgabe der Erfindung, unterschiedliche Fehlerquellen bei einer Abweichung eines Messwerts von einer modellbasierten Vorhersage zu finden bzw. die Genauigkeit einer modellbasierten Vorhersage zu verbessern. Dabei soll die modellbasierte Vorhersage auch den Einfluss von zumindest zwei Betriebsparametern des Prozesses berücksichtigen, ohne jedoch eine - in der Praxis nicht vorhandene - genaue Kenntnis der Betriebsparameter vorauszusetzen.

Die Aufgabe wird bei einem Verfahren der eingangs angeführten Art erfindungsgemäß dadurch gelöst, dass
- die Ausgangszusammensetzung gemessen oder vorgegeben wird,
- ausgehend von der (gemessenen oder vorgegebenen) Ausgangszusammensetzung und unter Variation der zumindest zwei Betriebsparameter eine Reaktionsfläche von erwarteten Produktzusammensetzungen simuliert wird, wobei jeder Punkt auf der Reaktionsfläche zumindest einer Konfiguration der Betriebsparameter entspricht,
- eine Produktzusammensetzung zumindest teilweise gemessen wird, und
- die zumindest teilweise gemessenen Produktzusammensetzung mit der Reaktionsfläche zur Erkennung von Mess- oder Simulationsfehlern, zur Erkennung einer Änderung der Ausgangszusammensetzung oder zur Ergänzung der gemessenen Produktzusammensetzung in Beziehung gesetzt wird.

Dementsprechend wird zur Lösung der genannten Aufgabe ein System zur Optimierung eines chemischen Verarbeitungsprozesses mit einer Produktanalyseeinheit zur Messung der Produktzusammensetzung eines Produkts des Verarbeitungsprozesses vorgeschlagen, wobei die Produktanalyseeinheit mit einer Auswerteeinheit verbunden ist, welche eingerichtet ist, ausgehend von einer, vorzugsweise mit einer ebenfalls mit der Auswerteeinheit verbundenen Ausgangsanalyseeinheit zur Messung der Ausgangszusammensetzung eines Ausgangsstoffs des Verarbeitungsprozesses, gemessenen oder vorgegebenen Ausgangszusammensetzung und unter Variation von zumindest zwei Betriebsparametern des Verarbeitungsprozesses eine Reaktionsfläche von erwarteten Produktzusammensetzungen zu simulieren und eine von der Produktanalyseeinheit gemessene Produktzusammensetzung mit der Reaktionsfläche in Beziehung zu setzen.

Der Erfindung liegt die Erkenntnis zugrunde, dass ein Modell des Verarbeitungsprozesses bei einer festgelegten (d.h. gemessenen oder vorgegebenen) Ausgangszusammensetzung mit einer durch Variation aller in das Modell einfließenden Betriebsparameter gebildeten Definitionsmenge zu einer Zielmenge von Produktzusammensetzungen führt, welche (bei einer Phase) nur zwei oder drei Freiheitsgrade aufweist, je nachdem ob sich das System im Gleichgewicht befindet oder nicht. Diese fundamentale Eigenschaft folgt aus der Gibbsschen Phasenregel, welche die Anzahl der physikalischen Freiheitsgrade eines Systems limitiert. Aufgrund dieser Eigenschaft des Verarbeitungsprozesses, d.h. weil sich der Einfluss sämtlicher Betriebsparameter auf wenige Freiheitsgrade reduziert, kann der Einfluss der Betriebsparameter auf die Produktzusammensetzung weitgehend von den Betriebsparametern entkoppelt werden. D.h. die Erfindung basiert auf der Erkenntnis, dass bei gegebener Ausgangszusammensetzung eines chemischen Verarbeitungsprozesses und nur durch Variation der Betriebsparameter (z.B. Druck und Temperatur des Prozesses) nicht beliebige Produktzusammensetzungen erzeugt werden können. Die daraus resultierenden Randbedingungen für alle möglichen Produktzusammensetzungen werden erfindungsgemäß zur Korrektur vollständiger Produktanalysen, zur Ergänzung unvollständiger Analysen oder zur Korrektur fehlerhafter Prozessmodelle verwendet; wobei es nicht nötig ist, alle auf die Produktzusammensetzung wirkenden Betriebsparameter zu berücksichtigen.

Der Einfluss der Betriebsparameter auf die Korrelation zwischen der Ausgangszusammensetzung und der Eingangszusammensetzung kann demzufolge bereits mit der Einführung von nur zwei Freiheitsgraden vollständig berücksichtigt werden. Anschaulich lässt sich dieser Zusammenhang zwischen einer Ausgangszusammensetzung und der Menge an - in Abhängigkeit vom Arbeitspunkt des Prozesses bzw. den Werten der Betriebsparameter - erzielbaren Produktzusammensetzungen durch eine so genannte Reaktionsfläche darstellen. Eine Reaktionsfläche entspricht der oben erwähnten Zielmenge des Verarbeitungsprozesses (bzw. dessen Modells) bei einer festgelegten Ausgangszusammensetzung und unter Variation der Betriebsparameter. Jede Konfiguration von Betriebsparametern entspricht einem eindeutig bestimmten Punkt auf der Reaktionsfläche. Die umgekehrte Zuordnung ist allerdings nicht eindeutig. Die Form und Lage der Reaktionsfläche ist durch die Ausgangszusammensetzung bestimmt, wobei der Rand der Reaktionsfläche durch die praktischen Limitierungen des Verarbeitungsprozesses (z.B. Betriebsfenster mit einem bestimmten Temperatur- und Druckbereich) festgelegt ist. Zur Auswertung der durch die Reaktionsfläche definierten Korrelation wird die zumindest teilweise gemessene Produktzusammensetzung mit der Reaktionsfläche in Beziehung gesetzt, d.h. es wird - anschaulich gesprochen - die Lage der gemessenen Produktzusammensetzung im Produktraum relativ zur Reaktionsfläche ermittelt.

Die Berechnung/Simulation der Reaktionsflächen kann z.B. unter Anwendung der PCA (Principal Component Analysis, Hauptkomponentenanalyse) durchgeführt werden. Es sollte jedoch jede dimensionsreduzierende Methode anwendbar sein.

Als Eingangsdaten der PCA dienen die per Simulation bzw. per Messung verfügbaren möglichen Zusammensetzungen des Produktes (z.B. Spaltgases) für eine Ausgangszusammensetzung. Man variiert die in Frage kommenden Betriebsparameter des Prozesses über das Betriebsfenster (Operating Window), wobei mindestens 2 für Gleichgewichtszustände, bzw. 3 Betriebsparameter für Reaktionen die zum Simulations- bzw. Messzeitpunkt nicht im Gleichgewichtszustand enden, nötig sind. Dies gilt für Reaktionen in einer Phase, sollten mehrere Phasen beteiligt sein, kommen entsprechend der Gibbschen Phasenregel noch weitere Freiheitsgrade bzw. Betriebsparameter hinzu. Zur Simulation können je nach Anwendungsgebiet übliche Prozesssimulationen, z.B. in Form von entsprechenden Softwarepaketen, verwendet werden. Bei einer Anwendung bei Steamcrackern kann dies beispielsweise eines der Produkte "HYSYS" der Firma Aspen Technology Inc., "Petrosim" der Firma KBC Advanced Technologies plc und "SPYRO" der Firma Technip Benelux B.V. sein.

Jedem Betriebspunkt im Operating Window kann so eine Produktzusammensetzung zugeordnet werden. Die möglichen Produktzusammensetzungen werden zu einer Matrix zusammengefasst, wobei jede Spalte der Matrix die Anteile einer Produktkomponente in Abhängigkeit der zugehörigen Betriebspunkte darstellt und die Reihen der Matrix jeweils einem Betriebspunkt entsprechen.

Diese n×m-dimensionale (n = Anzahl der Produktkomponenten; m = Anzahl der simulierten Betriebspunkte) Matrix wird nun mittels PCA auf eine Fläche mit 2 bzw. ein Volumen (bzw. Membran) mit 3 Freiheitsgraden abgebildet. In der Regel wird diese 2- bzw. 3-dimensionale Abbildung mittels PCA als "Scores" bezeichnet. Die PCA liefert zwar mehr als nur 2 oder 3 Score-Komponenten, aber mehr sind entsprechend der Gibbschen Phasenregel nicht nötig.

Einem Punkt auf der Score Fläche bzw. Membran können mehrere Betriebspunkte zugewiesen werden, aber nur eine mögliche Produktzusammensetzung. Diese Score Fläche/Membran ist die Reaktionsfläche bzw. das Reaktionsvolumen.

Zur Vereinfachung kann prinzipiell auch jede beliebige Kombination von Produktkomponenten gegeneinander im 3-dimensionalen Raum aufgetragen werden. Es wird immer eine Reaktionsfläche bzw. Volumen entstehen. Dies gilt ebenso für Abbildungen der Produktkomponenten, sofern kein neuer Freiheitsgrad hinzugefügt wird. Besteht das Produkt aus z.B. 5 Komponenten K1 bis K5, so spielt es keine Rolle ob man K1 vs. K2 vs. K3 oder log(K1) vs. K2^2×K4^3 vs. Exp(K3+K5) aufträgt, es wird sich immer eine Reaktionsfläche bzw. Volumen ergeben, da nicht mehr wahre Freiheitsgrade vorhanden sind.

Der Vorteil der PCA gegenüber dem vereinfachten Ansatz ergibt sich dadurch, dass in der Scores Darstellung alle Produktkomponenten eingehen. Ein Punkt der Fläche wird durch eine Vielzahl von Produktkomponenten/Messungen bestimmt und ist im statistischen Sinn daher viel robuster und genauer, als wenn nur einzelne Produktkomponenten gegeneinander aufgetragen werden. Wobei die Mess- bzw. Simulationsgenauigkeit einzelner Komponenten natürlich mittels Gewichtung eingehen kann.

Obiges Prozedere lässt sich auch für Variationen von Ausgangs- bzw. Einsatzzusammensetzungen durchführen, wobei in Folge ein 3-dimensionales Reaktionsvolumen entsteht.

Ob die Ausgangszusammensetzung gemessen oder vorgegeben (z.B. eingegeben oder als bekannt angenommen) wird, hängt von der jeweiligen Anwendung des verstehend erläuterten Verfahrens ab. Eine Messung der Ausgangszusammensetzung ist z.B. erforderlich, wenn man die Differenz zwischen der (gemessenen) Ausgangszusammensetzung und dem was das Simulationsmodell als Ausgangszusammensetzung "benötigt", um die gemessenen Spaltgaskomponenten auf eine entsprechende (nämlich z.B. die gemessene) Reaktionsfläche abzubilden, berechnen will. In diesem Fall ist die Ausgangszusammensetzung a priori unbekannt und muss per Messung ermittelt werden, um die Korrekturfunktion der Ausgangszusammensetzung zu bestimmen. Dies ist ein wichtiger Anwendungsfall - aber nicht der einzige.

In einem anderen Anwendungsfall ist die Ausgangszusammensetzung bekannt, muss aber zwecks Erhöhung der Vorhersagegenauigkeit mittels Korrekturfunktion der Ausgangszusammensetzung angepasst werden. Die könnte z.B. für einen Ausgangsstoff wie reines Ethan, bei dem es keinen Sinn macht, die Ausgangszusammensetzung anzuzweifeln oder zu messen, weil sie genau bekannt ist (auch ohne Messung) und sich verfahrenstechnisch auch nicht signifikant ändern kann. Trotzdem kann mit Hilfe des obigen Modells - unter Variation der vorgegebenen Ausgangszusammensetzung - und der Reaktionsflächen berechnet werden, wie die Ausgangszusammensetzung hätte aussehen müssen, damit die Simulation zu der gemessenen Reaktionsfläche geführt hätte. Daraus wiederum lässt sich die Differenz zwischen der bekannten und der so durch Variation ermittelten Ausgangszusammensetzung berechnen, um die gewünschte Vorhersagegenauigkeit zu erhalten.

Es ist aber auch denkbar, dass die Ausgangszusammensetzung unbekannt ist und per Simulation iterativ ermittelt wird: gibt es bei einem Einsatz z.B. nur wenige mögliche Komponenten des Ausgangsstoffs, z.B. Ethan, Propan und Butan, und bewirken diese jeweils starke Verschiebungen der Reaktionsflächen und liegen die gemessenen Produktzusammensetzungen gut verteilt auf einer Reaktionsfläche, so kann eine eindeutige Ausgangszusammensetzung ausreichend genau berechnet werden. Aufgrund des Umwegs über die Rekonstruktion einer Reaktionsfläche können Betriebsparameterfehler das Ergebnis nicht verfälschen. In diesem Zusammenhang liegt eine gute Verteilung der gemessenen Produktzusammensetzungen auf der Reaktionsfläche dann vor, wenn diese in Relation zum Messfehler der einzelnen Messungen ausreichend weit gestreut sind, um die Menge an möglichen Reaktionsflächen (d.h. welche die gemessenen Produktzusammensetzungen bei bekanntem Messfehler am besten wiedergeben bzw. welche im Sinne der Fehlerverteilung am wahrscheinlichsten sind) einzugrenzen, und nicht eng bei einander liegen. Der Zusammenhang zwischen der Verteilung und den Messfehlern lässt sich anhand eines Beispiels leicht nachvollziehen: um eine ebene Fläche zu beschreiben, werden bekanntlich zumindest drei Punkte (der Produktzusammensetzung) benötigt, z.B. (in m% von drei Produktkomponenten angegeben) Punkt 1: 80/5/15, Punkt 2: 80/4.9/15.1 und Punkt 3: 79.9/5.1/15. Beträgt der Messfehler 0 m%, lässt sich damit die Reaktionsfläche exakt definieren. Beträgt der Messfehler jedoch 0.1 m%, dann gibt es bereits eine Vielzahl von Möglichkeiten für die gesuchte Reaktionsfläche; würden die drei Punkte bei z.B. Punkt 1: 80/5/15, Punkt 2: 70/10/20 und Punkt 3: 50/30/20 liegen, dann wäre ein Fehler von 0.1 m% weniger signifikant. Liegen die Ausgangszusammensetzungen eng beieinander, so sind die zugehörigen Betriebspunkte naturgemäß meist ebenso wenig unterschiedlich. Darum lohnt es sich, z.B. ofenübergreifend zu korrigieren, da die Betriebspunkte vieler Öfen in der Regel gut verteilt (d.h. ausreichend unterschiedlich) sind.

Schließlich besteht eine möglich Anwendung, welche ohne eine Messung der Ausgangszusammensetzung auskommt darin, entweder lediglich Änderungen an einer Ausgangszusammensetzung zu erkennen oder eine gemessene Produktzusammensetzung zu überprüfen und zu plausibilisieren. In diesem Fall genügt es, zu überprüfen ob die gemessenen Produktzusammensetzungen auf einer Fläche (bzw. innerhalb einer Membran) liegen. Befinden sich z.B. die letzten 50 Messpunkte auf einer Fläche und der nächste Punkt weicht statistisch signifikant von dieser Fläche ab, dann hat sich entweder die Ausgangszusammensetzung oder die Produktprobennahme bzw. die Produktanalyse geändert. Analysieren z.B. zwei Gaschromatographen (GC) das Spaltgas zweier Öfen und bekommen diese beiden Öfen den gleichen Einsatz, so müssen beide GCs die gleichen Reaktionsflächen erzeugen - falls nicht, folgt daraus, dass die Spaltgasprobennahme bzw. -analyse signifikant fehlerhaft ist. Werden z.B. zwei Spaltgase von zwei Öfen mittels des selben GC gemessen (multiple stream GCs, oder NMR-Analysatoren usw.), so kann der Fehler hingegen nicht vom GC kommen, da es der selbe GC ist. Die Kenntnis der Ausgangszusammensetzung ist daher in diesen Fällen nicht einmal näherungsweise nötig und es wird lediglich implizit durch die empirisch ermittelte bzw. geschätzte Reaktionsfläche eine Ausgangszusammensetzung vorgegeben (die jedoch in diesem Fall weder gemessen noch explizit angegeben oder berechnet werden muss).

Im Zusammenhang mit dem erfindungsgemäßen System hat es sich als vorteilhaft herausgestellt, wenn die Auswerteeinheit ein Simulationsmodul zur Simulation der Reaktionsfläche und ein Vergleichsmodul, welches zur Erkennung eines Fehlers der Ausgangsanalyseeinheit, der Produktanalyseeinheit und/oder des Simulationsmoduls sowie zur Ermittlung einer Korrektur zur Behebung des Fehlers eingerichtet ist, wobei das Korrekturmodul zur Übermittlung der ermittelten Korrektur vorzugsweise mit der Ausgangsanalyseeinheit, der Produktanalyseeinheit und/oder dem Simulationsmodul verbunden ist. Durch diesen Aufbau kann das Simulationsmodul leicht ausgetauscht bzw. gegebenenfalls verschiedene Simulationsmodule verwendet werden. Dies entspricht der vielseitigen Anwendbarkeit der vorliegenden Erfindung auf verschiedenartigste Verarbeitungsprozesse, wobei lediglich das Simulationsmodul spezifisch auf den jeweils optimierten Prozess, d.h. insbesondere die ablaufenden Reaktionen, zugeschnitten ist.

Wenn die Reaktionsfläche eine höchstens dreidimensionale, vorzugsweise nur zweidimensionale, Menge von Produktzusammensetzungen ist, wobei die Anzahl der Dimensionen der Reaktionsfläche der Anzahl der physikalischen Freiheitsgrade des Verarbeitungsprozesses entspricht, kann die Korrelation zwischen der Ausgangszusammensetzung und der Produktzusammensetzung besonders genau angegeben werden, was die bei der Auswertung der gemessenen Zusammensetzungen verfügbare Information erhöht. Der bevorzugte Fall einer zweidimensionalen Menge betrifft Gleichgewichtsreaktionen, bei denen das kontinuierlich aufrecht erhaltene Gleichgewicht einen Freiheitsgrad konsumiert. Abseits des Gleichgewichts, z.B. wenn im Verarbeitungsprozess nicht genügend Zeit zur Erreichung des Gleichgewichts vorhanden ist, kommt maximal ein dritter Freiheitsgrad, z.B. die Gibbs-Energie G (wegen dG ≠ 0 in diesem Fall), hinzu. Auch wenn der Begriff der Fläche für dreidimensionale Mengen mathematisch nicht korrekt ist, wird hier der Einfachheit halber immer von Reaktionsflächen gesprochen, womit im Allgemeinen sowohl zweidimensionale Flächen als auch (meist dünne) dreidimensionale Körper (z.B. Membranen oder Schalen) gemeint sind.

Insbesondere hat es sich als vorteilhaft herausgestellt, wenn eine Abweichung der zumindest teilweise gemessenen Produktzusammensetzung von der Reaktionsfläche ermittelt wird und die ermittelte Abweichung zur Erkennung eines Fehler bei der Messungen der Ausgangszusammensetzung, bei der Messung der Produktzusammensetzung oder in der Simulation verwendet wird. Eine Abweichung bedeutet im Wesentlichen, dass die gemessene Produktzusammensetzung nicht in der Reaktionsfläche liegt. Quantitativ kann die Abweichung beispielsweise als ein geometrischer Abstand zwischen der Produktzusammensetzung und der Reaktionsfläche angegeben werden, wobei ein einen vorgegebenen Schwellwert überschreitender Abstand auf einen Fehler hindeutet.

In diesem Zusammenhang kann günstigenfalls ein Korrekturwert für die Messung der Produktzusammensetzung aus der ermittelten Abweichung ermittelt werden, wobei vorzugsweise aus mehreren gemessenen Produktzusammensetzungen eine Abstandsfunktion zwischen diesen und der Reaktionsfläche ermittelt wird, welche Abstandfunktion einem von der gemessenen Produktzusammensetzung abhängigen Korrekturwert entspricht. Die Ermittlung eines Korrekturwerts für die Messung der Produktzusammensetzung bietet sich besonders an, wenn die Ursache für einen Messfehler bei der Messung der Produktzusammensetzung liegt oder zumindest angenommen wird. Ein Grund, eine solche Ursache anzunehmen kann z.B. sein, dass der Ausgangsstoff in mehreren parallelen Verarbeitungsprozessen verarbeitet wird und die gemessenen Produktzusammensetzungen der beiden Prozesse auf unterschiedlichen Reaktionsflächen liegen. Ebenso ist ein Messfehler bei der Messung der Produktzusammensetzung anzunehmen, wenn bei gleich bleibendem Ausgangsstoff eine Verschiebung der gemessenen Produktzusammensetzung festgestellt wird.

Falls beispielsweise bei unabhängigen Messungen der Ausgangszusammensetzung und der Produktzusammensetzung, insbesondere bei unabhängig ablaufenden Verarbeitungsprozessen, systematische Abweichungen festgestellt werden, kann vorteilhaft ein Fehler in der Simulation korrigiert werden, indem zumindest ein Modellparameter eines der Simulation zugrunde liegenden Modells des Verarbeitungsprozesses so verändert wird, dass die gemessene Produktzusammensetzung in einer auf Basis des zumindest einen veränderten Modellparameters aktualisierten Reaktionsfläche liegt.

In diesem Zusammenhang ist es besonders vorteilhaft, wenn bei einem Verarbeitungsprozess mit mehreren Reaktoren, wobei die gemessene Produktzusammensetzung die Gesamtheit der Produkte der Reaktoren betrifft, ein einem einzelnen Reaktor zugeordneter Modellparameter oder ein mehreren Reaktoren zugeordneter Modellparameter so geändert wird, dass eine lokal möglichst glatte Reaktionsfläche erzielt wird. Falls es mehrere unterschiedliche Reaktoren gibt, die unterschiedliche Betriebspunkte (oder sogar Einsätze, z.B. ein Coil bekommt einen Haupt-Einsatz und einen Co-Einsatz, variiert diese Mischung durch Mess-/Regelungsfehler, ist auch der Einsatz variabel) aufweisen können und die Ausbeute nicht pro Reaktor sondern nur in Summe über alle Reaktoren gemessen wird, kann es durch die nichtlineare Eigenschaft der Reaktionsflächen den Anschein haben, dass die Reaktionsfläche nicht glatt ist - was wiederum ein Hinweis darauf ist, dass die Reaktoren ungleich betrieben werden. Lokale Unebenheiten in der Reaktionsfläche (d.h. eine weniger glatte Reaktionsfläche) entsprechen im Allgemeinen weniger der Realität, da Unebenheiten auf zusätzliche Freiheitsgrade hindeuten, die jedoch physikalisch nicht vorhanden sind. Solche scheinbaren Unebenheiten können beispielsweise durch Nichtlinearitäten bei der Messung einer gemischten Produktzusammensetzung mit Produkten aus mehreren unabhängigen Reaktoren auftreten. In diesem Fall sollte ein einem einzelnen Reaktor zugeordneter Modellparameter anstelle eines globalen Modellparameters zur Korrektur angepasst werden. Wenn mehrere Messungen der Produktzusammensetzung vorhanden sind, können bei einer Anpassung der Modellparameter an die Messungen, welche anschaulich einer Variation der Form und Lage der Reaktionsfläche entspricht, selbstverständlich auch zwei, drei oder mehr Modellparameter gleichzeitig angepasst werden, um die gewünschte Variation der Reaktionsfläche zu erzielen. Auf diese Weise kann das Modell optimal parametriert werden, um genaue Vorhersagen der Produktzusammensetzung zu ermöglichen. Z.B. seien zwei Spaltcoils gegeben, welche beide auf einen Zielwert von COT 840 °C betrieben werden. Durch Fehler im System kommt es aber dazu, dass die vorgegeben 840 °C in Wirklichkeit schwanken/schwingen, bzw. einen anderen Wert als gewünscht aufweisen. Dadurch verschieben sich die Ausbeutepunkte der Spaltcoils auf der Reaktionsfläche. Nachdem ein Analysator nur die Mischung analysiert und die Mischung nicht auf der Reaktionsfläche liegen muss, kann das den Anschein erwecken, dass die Reaktionsfläche nicht glatt ist. Weiß man z.B. wie die einzelnen Spaltcoils schwingen oder gibt es zwischen den Spaltcoils konstante Anteile der Abweichungen in den Betriebsparametern, können Korrekturparameter gewählt und so angepasst werden, sodass diese Unebenheiten kleiner werden.

Eine weitere vorteilhafte Anwendung des durch die Reaktionsfläche hergestellten Zusammenhangs zwischen der Ausgangszusammensetzung und der Produktzusammensetzung besteht darin, dass ein einer unvollständig gemessenen Produktzusammensetzung nächstliegender Punkt der Reaktionsfläche ermittelt wird und die unvollständig gemessenen Produktzusammensetzung durch die diesem Punkt entsprechende, erwartete Produktzusammensetzung ergänzt wird. Indem einzelne Produktkomponenten nicht gemessen werden, kann die Messung einfacher und kostengünstiger durchgeführt werden und es können - je nach Auswahl der tatsächlich gemessenen Komponenten - schnellere Analysetechniken eingesetzt werden, welche in der Folge auch ein rascheres Feedback zur Prozesssteuerung ermöglicht.

Vorzugsweise kann bei der Ergänzung einer unvollständig gemessenen Produktzusammensetzung durch die Ermittlung des nächstliegenden Punkts der Reaktionsfläche eine den einzelnen Komponenten der gemessenen Produktzusammensetzung zugeordnete Messgenauigkeit berücksichtigt werden, indem die Abstände der einzelnen Komponenten mit der Messgenauigkeit gewichtet werden. Eine Gewichtung mit der Messgenauigkeit entspricht einer Skalierung der Differenzen der jeweiligen Komponenten z.B. mit dem inversen zugeordneten Messfehler. Diese Skalierung wirkt sich auf die Wahl des nächstliegenden Punkts der Reaktionsfläche aus. Anschaulich ist die gemessene Produktzusammensetzung hinsichtlich einer Komponente mit größerem zugeordneten Messfehler beweglicher als hinsichtlich einer Komponente mit kleinerem zugeordneten Messfehler. Der Extremfall einer nicht gemessenen Komponente entspricht dabei einer unendlichen Beweglichkeit und bedeutet, dass diese Produktkomponente bei der Ermittlung des nächstliegenden Punkts nicht berücksichtigt wird (der gewichtete Abstand ist immer Null).

Die Erfindung wird nachfolgend anhand von besonders bevorzugten Ausführungsbeispielen, auf die sie jedoch nicht beschränkt sein soll, und unter Bezugnahme auf die Zeichnungen noch weiter erläutert. In den Zeichnungen zeigen dabei im Einzelnen:
Fig. 1 einen groben schematischen Überblick über den Verarbeitungsprozess;
Fig. 2 schematisch ein System zur Optimierung des Verarbeitungsprozesses;
Fig. 3 eine orthogonale 3D-Darstellung einer Reaktionsfläche von erwarteten Produktzusammensetzungen;
Fig. 4a-d zwei Reaktionsflächen gemäß Fig. 3 für verschiedene, ähnliche Ausgangszusammensetzung in einer axonometrischen Ansicht (Fig. 4a) und drei Normalprojektionen (Fig. 4b-d);
Fig. 5 zwei Reaktionsflächen gemäß Fig. 3 für völlig verschiedene Ausgangszusammensetzung;
Fig. 6 eine Reaktionsfläche gemäß Fig. 3 für verschiedene Verarbeitungseinheiten;
Fig. 7 eine Reaktionsfläche gemäß Fig. 3 mit drei verschiedenen Anwendungsfällen; und
Fig. 8a-d eine Reaktionsfläche gemäß Fig. 3 mit einem vierten Anwendungsfall in einer axonometrischen Ansicht (Fig. 8a) und drei Normalprojektionen (Fig. 8b-d).

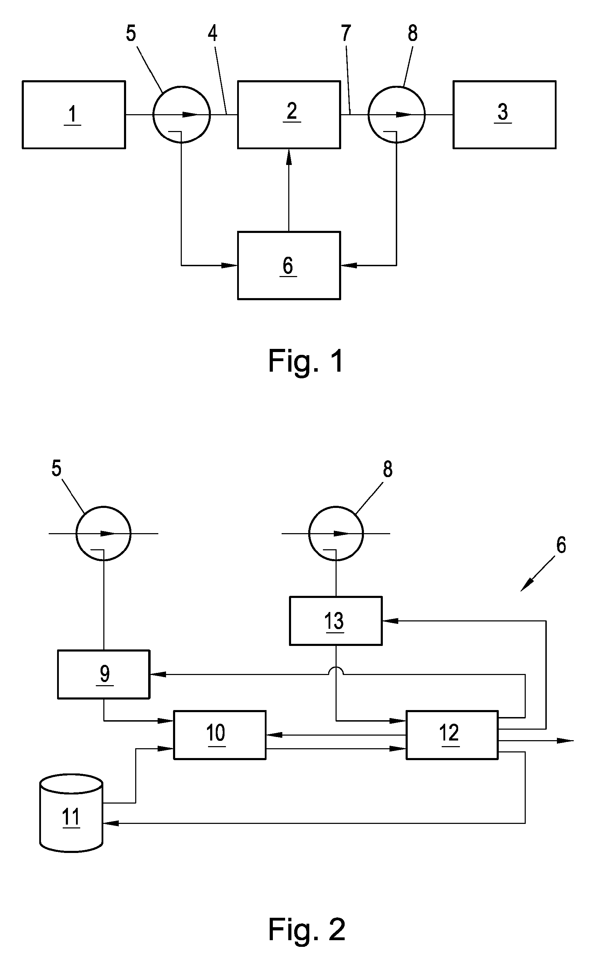

Fig. 1 zeigt ein Blockschaltbild eines allgemeinen Verarbeitungsprozesses, mit einem Ausgangsstoff 1, welcher in einer Verarbeitungseinheit 2 zu einem Produkt 3 verarbeitet wird. Die Verarbeitungseinheit 2 kann beispielsweise ein Reaktor, insbesondere ein Ofen, sein. Der Ausgangsstoff 1 und das Produkt 3 sind im Allgemeinen jeweils flüssige oder gasförmige Zusammensetzungen mehrerer chemischer Komponenten, wobei das Produkt 3 vorzugsweise eine Zusammensetzung von zumindest drei Komponenten ist. Mit einer Zuleitung 4 der Verarbeitungseinheit 2 zur Zuführung des Ausgangsstoffs 1 ist eine Ausgangsanalyseeinheit 5 verbunden, wobei die Verbindung eine Probennahme aus der Zuleitung 4 erlaubt. Die Ausgangsanalyseeinheit 5 ist eingerichtet, eine Ausgangszusammensetzung des Ausgangsstoffs 1 zu ermitteln. Die Ausgangsanalyseeinheit 5 umfasst beispielsweise eine Gaschromatographen zur Analyse einer aus der Zuleitung 4 entnommenen Probe des Ausgangsstoffs 1. Weiters ist die Ausgangsanalyseeinheit 5 mit einer Auswerteeinheit 6 verbunden, so dass die ermittelte Ausgangszusammensetzung an die Auswerteeinheit 6 übertragen werden kann. Analog zur Ausgangsanalyseeinheit 5 ist mit einer Ableitung 7 zur Weiterleitung des Produkts 3 der Verarbeitungseinheit 2 eine Produktanalyseeinheit 8 verbunden. Auch hier besteht die Verbindung zwischen der Ableitung 7 und der Produktanalyseeinheit 8 darin, dass zumindest eine Probennahme möglich ist. Die Produktanalyseeinheit 8 ist zu Ermittlung einer Produktzusammensetzung des Produkts 3 eingerichtet und umfasst beispielsweise einen Gaschromatographen zur Analyse einer aus der Ableitung 7 entnommenen Probe des Produkts 3. Dem Fachmann ist ohne Weiteres geläufig, dass die Analyseeinheiten 5, 8 je nach Zustand der zu analysierenden Stoffe 1, 3 weitere Vorrichtungen, z.B. Abkühlungs- oder Kondensationseinrichtungen oder sonstige Probenaufbereitungseinrichtungen, umfassen können. Zur Übertragung der ermittelten Produktzusammensetzung ist auch die Produktanalyseeinheit 8 mit der Auswerteeinheit 6 verbunden. Die Auswerteeinheit 6 erhält demzufolge sowohl eine ermittelte Ausgangszusammensetzung als auch einer ermittelte Produktzusammensetzung. Die Auswerteeinheit 6 umfasst ein Modell des in der Verarbeitungseinheit 2 ablaufenden Verarbeitungsprozesses und ist eingerichtet, zumindest eine Konsistenzprüfung der erhaltenen Produktzusammensetzung mit der erhaltenen Ausgangszusammensetzung unter Anwendung des Modells durchzuführen. Insbesondere ist die Auswerteeinheit 6 eingerichtet, der Verarbeitungseinheit 2 korrigierte Messwerte und/oder korrigierte Modellparameter (bzw. jeweils entsprechende Korrekturwerte) zur optimalen Steuerung des Verarbeitungsprozesses zu übermitteln.

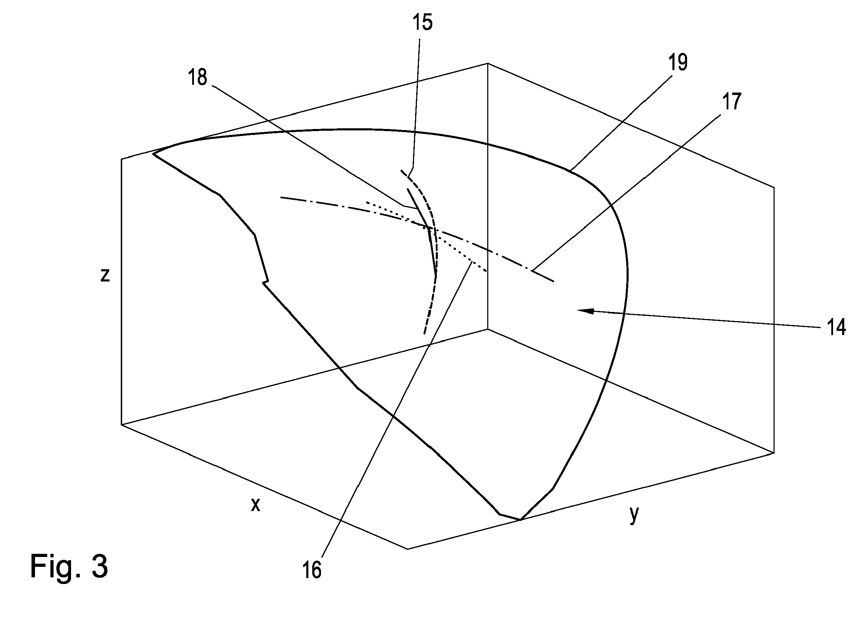

In Fig. 2 ist der Aufbau der Auswerteeinheit 6 dargestellt. Die von der Ausgangsanalyseeinheit 5 ermittelte Ausgangszusammensetzung wird zunächst in einem ersten Korrekturmodul 9 anhand von gespeicherten Korrekturwerten korrigiert. Die korrigierte Ausgangszusammensetzung wird an ein Simulationsmodul 10 übermittelt. Das Simulationsmodul 10 berechnet ausgehend von der empfangenen Ausgangszusammensetzung und von in einer Betriebsdatenbank 11 abgelegten Modellparametern bzw. Randbedingungen für etwaige Betriebsparameter des Verarbeitungsprozesses eine Reaktionsfläche von erwarteten Produktzusammensetzungen. Diese Berechnung bzw. die Simulation der Reaktionsfläche kann durch Einsetzen in ein analytisches Modell, durch Ausführung einer numerischen Simulation oder durch Abrufen und/oder Interpolation von empirisch ermittelten Ergebnissen erfolgen. Bei einer Anwendung zur Optimierung eines Steamcrackers kann beispielsweise das Modell SPYRO der Fa. Technip Benelux B.V. verwendet werden. Aus den so erhaltenen einzelnen Produktzusammensetzungen wird die Reaktionsfläche z.B. mittels PCA (s. oben) berechnet, wobei zwecks Verbesserung der Robustheit und Genauigkeit möglichst viele Spaltgaskomponenten Verwendung finden sollen, d.h. auch wenn grundsätzlich nur drei beliebige Komponenten gegeneinander aufgetragen und daraus die Reaktionsfläche ermittelt werden könnte, kann mit mehr als drei, insbesondere mit mehr als vier, Komponenten eine höhere Robustheit und Genauigkeit bei der Berechnung der Reaktionsfläche erzielt werden. Die simulierte Reaktionsfläche wird an ein Vergleichsmodul 12 übermittelt, welches außerdem von einem zweiten Korrekturmodul 13 eine, auf Basis der Messung der Produktanalyseeinheit 8, korrigierte Produktzusammensetzung erhält. Das Vergleichsmodul 12 ist zur Erkennung eines Fehlers der Ausgangsanalyseeinheit 5, der Produktanalyseeinheit 8 und/oder des Simulationsmoduls 10 sowie zur Ermittlung einer Korrektur zur Behebung des Fehlers eingerichtet, wie anhand der folgenden Diagramme (Fig. 3-8) genauer beschrieben wird. Außerdem ist das Vergleichsmodul 12 zur Anpassung der gespeicherten Korrekturwerte und/oder von Modellparametern mit den Korrekturmodulen 9, 13 sowie dem Simulationsmodul 10 verbunden.

Die Diagramme in Fig. 3, 4a, 5 bis 7 und 8a stellen jeweils eine (oder mehrere) Reaktionsfläche(n) 14 (14', etc.) in einem dreidimensionalen Koordinatensystem gemäß einer axonometrischen Projektion dar. Dabei ist an jeder Achse x, y, z eine Komponente der Produktzusammensetzung aufgetragen. Demzufolge werden Produktzusammensetzungen mit mehr als drei Komponenten in dieser Darstellung nur teilweise wiedergegeben, d.h. die Werte etwaiger weiterer Komponenten sind aus den Diagrammen jeweils nicht ablesbar. Die Reaktionsfläche 14 stellt einen Zusammenhang zwischen den dargestellten Produktkomponenten x, y, z her. Das in Fig. 3 abgebildete Beispiel entspricht der Reaktionsfläche eines Steamcrackers, wobei ausgehend von dem Ausgangsstoff Naphtha ein Steamcracking-Prozess simuliert wurde. Die Produktkomponenten x, y, z des resultierenden Spaltgases repräsentieren jeweils den Anteil (in Gewichtsprozent) an RohC4 (umfasst alle C4 Verbindungen), C2H4 bzw. C3H6. Im Allgemeinen können die Variablen x, y, z auch Abbildungen der Produktkomponenten sein, z.B. entsprechend den ersten drei Scores PCA (s. oben). Jeder Punkt auf der Reaktionsfläche 14 entspricht einer bestimmten Produktzusammensetzung. Dies gilt auch für nicht dargestellte Komponenten, d.h. der Anteil einer nicht dargestellten Produktkomponente w ist für jeden Punkt auf der Reaktionsfläche ebenso festgelegt und im Allgemeinen für verschiedene Punkte unterschiedliche hoch.

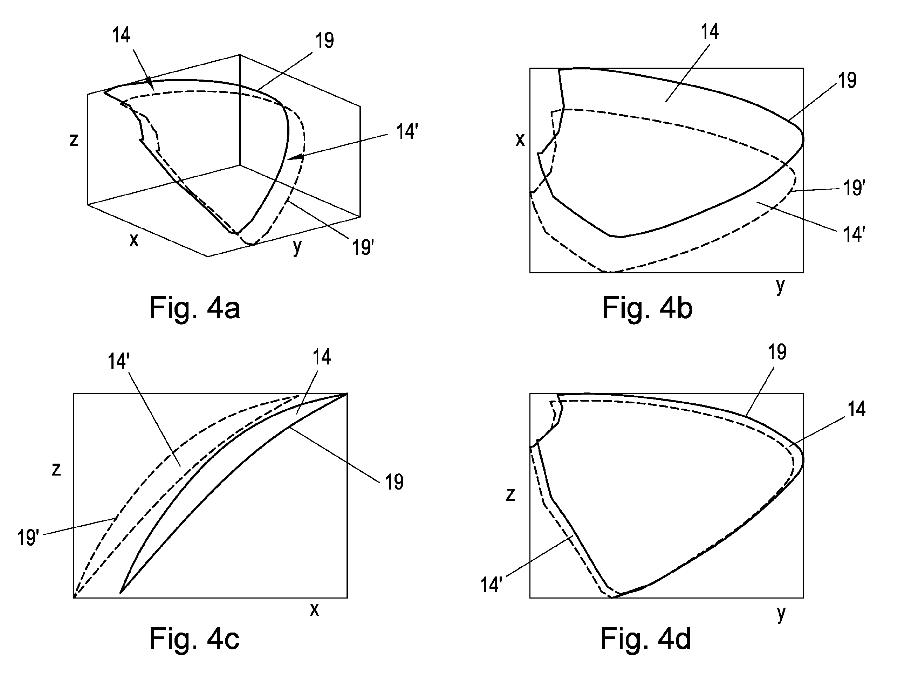

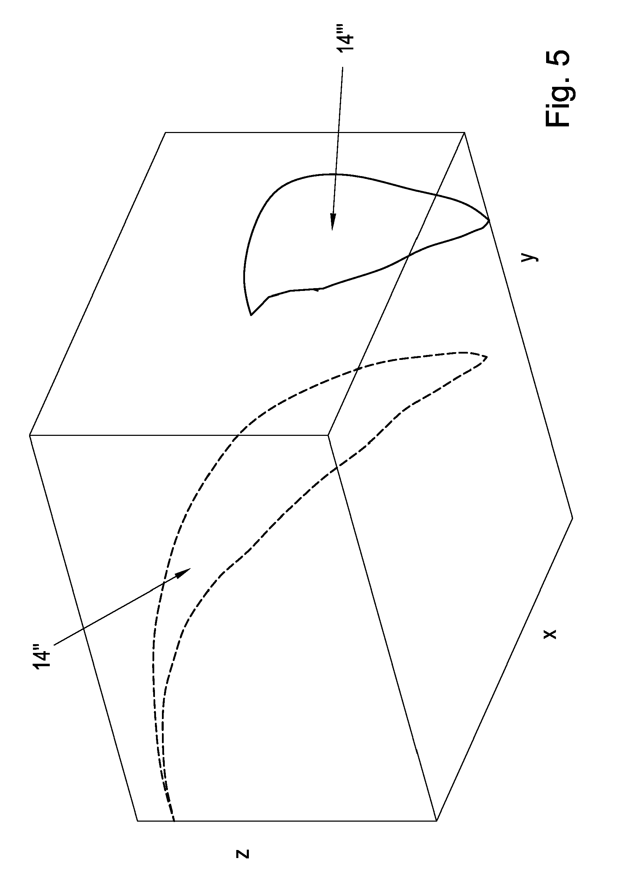

Die beiden Freiheitsgrade auf der Reaktionsfläche ergeben sich aus der Variation von zumindest zwei Betriebsparametern des Verarbeitungsprozesses. Aber auch wenn mehr als zwei Betriebsparameter variiert werden, steigt die Anzahl der Freiheitsgrade auf der Reaktionsfläche 14 nicht weiter an, d.h. unabhängig davon ob zwei oder mehr Betriebsparameter variiert werden, bleibt die Anzahl der Freiheitsgrade auf der Reaktionsfläche 14 für Gleichgewichtszustände zwei. Demzufolge ist die Zuordnung der Punkte auf der Reaktionsfläche zu den entsprechenden Betriebsparametern mehrdeutig; jeder Punkt kann durch eine Vielzahl an Kombinationen der Betriebsparameter erreicht werden. Diese Beschränkung der Freiheitsgrade der Reaktionsfläche 14 entspricht einem Grundsatz der Thermodynamik: da sämtliche Ausgangs- und Produktkomponenten durch eine oder mehrere Austauschreaktionen gekoppelt sind, ist die Anzahl der Freiheitsgrade im Gleichgewicht gemäß der Gibbsschen Phasenregel auf zwei Zustandsgrößen limitiert (z.B. Druck und Temperatur). In Fig. 3 ist die Variation von vier Betriebsparametern bei der Simulation der Reaktionsfläche 14 durch entsprechende Pfade 15, 16, 17, 18 auf der Reaktionsfläche 14 angedeutet. Jeder Pfad 15, 16, 17, 18 entspricht dabei einem Betriebsparameter, wobei im vorliegenden Beispiel eine Spaltgastemperatur 16 an einem Spaltrohr-Austritt (Coil-Outlet-Temperature; COT), ein Spaltgasdruck 18 am Spaltrohr-Austritt (COP), eine Einsatzmenge 17 an zu spaltenden Kohlenwasserstoffen an einem Spaltrohr-Eintritt (FLOWR) und ein Verhältnis 15 eines Prozessdampf-Volumens zur Einsatzmenge (D/KW) als Betriebsparameter verwendet, d.h. zur Simulation der Reaktionsfläche 14 variiert wurden. Manche der Pfade 15, 18 bzw. 16, 17 verlaufen beinahe parallel, so dass unmittelbar ersichtlich ist, dass z.B. eine Änderung der COT 16 durch eine komplementäre Änderung der FLOWR 17 im Wesentlichen kompensiert werden kann. Die Form und Lage der Fläche ist unabhängig von den Betriebsparameters und resultiert aus der Ausgangszusammensetzung, von der ausgehend die Produktzusammensetzungen simuliert wurden, d.h. verschiedene Ausgangszusammensetzungen führen zu unterschiedlichen Reaktionsflächen. Solche verschiedenen Reaktionsflächen 14, 14' für unterschiedliche Ausgangszusammensetzungen sind schematisch in Fig. 4a-d und Fig. 5 dargestellt.

Die Grenze 19 der Reaktionsfläche 14 ist durch ein Betriebsfenster ("operating window") des Verarbeitungsprozesses bzw. der Verarbeitungseinheit 2 festgelegt. Das Betriebsfenster definiert dabei für jeden Betriebsparameter einen Bereich, in welchem der Betriebsparameter zur Simulation der Reaktionsfläche variiert wird. Die Reaktionsfläche 14 ist somit eine vollständige Repräsentation des Modells des Verarbeitungsprozesses bei einer vorbestimmten Ausgangszusammensetzung und innerhalb eines begrenzten Betriebsfensters.

Bei der Erstellung eines Betriebsfensters, das auf die Reaktionsfläche abgebildet wird, kann beispielsweise wie folgt vorgegangen werden:
- Wahl der zu variierenden Betriebsparameter

Im Falle des thermischen Crackens sind z.B. mindestens 3 Freiheitsgrade/Betriebsparameter nötig. Beispielsweise wählt man 4 Freiheitsgrade (COT, D/KW, FLOWR, COP), damit in Folge gezeigt wird, dass definitiv nicht mehr als 3 wahre Freiheitsgrade vorhanden sind. Hierbei ist es zielführend (aber nicht zwingend erforderlich) Betriebsparameter mit einer möglichst hohen Auswirkung auf die Ausbeute zu wählen.
- Wahl des zu betrachtenden Bereiches, wobei der Bereich je nach Anwendung gewählt wird:

Ist das Ziel z.B. den unterschiedlichen Einsatz zu vergleichen, wird man einen möglichst großen Bereich wählen z.B. COT 800-900 °C, D/KW 0.2 - 1.0, FLOWR 12- 24 t/h, COP 1.2 - 2.2 bar, ist das Ziel möglichst effizient (CPU-Ressourcen schonend) eine Abweichung eines gemessenen Ausbeutepunktes zu einer Reaktionsfläche zu bestimmen, wird man einen Bereich um den dem Ausbeutepunkt zugehörigen Betriebspunkt wählen. Z.B. wurde der Ausbeutepunkt mit COT 850 °C, D/KW 0.6, FLOWR 20 t/h, COP 1.9 bar erzeugt, dann wird ein Bereich von z.B. COT 840 - 860 °C, D/KW 0.4 - 0-8, FLOWR 18-22 t/h, COP 1.7 - 2.1 bar sinnvoll sein.
- Rastern der Betriebsparameter:

Jeder Betriebsparameter wird nun im betrachteten Bereich gerastert, d.h. die Betriebsparameter werden im betrachteten Bereich variiert: z.B. COT 840 / 850 / 860 °C, D/KW 0.4 / 0.6 / 0.8, FLOWR 18 / 20 / 22 t/h, COP 1.7 / 1.9 / 2.1 bar. Je stärker nichtlinear die Abhängigkeit eines Betriebsparameters auf die Ausbeute im betrachteten Bereich ist, umso mehr Variationen wird man wählen.
- Kombination jeder Variation eines Betriebsparameters mit denen von allen anderen:

Im hier angeführten Beispiel werden 4 Betriebsparameter jeweils 3 mal variiert, d.h. es gibt in Summe 4^3 = 81 Kombinationsmöglichkeiten = Betriebspunkte. Es spielt hierbei keine Rolle, ob die Abstände zwischen den Betriebspunkten äquidistant sind, man könnte ebenso zufällig verteilte Betriebspunkte wählen, sofern diese ausreichend breit und gleichmäßig auf der Reaktionsfläche verstreut sind, um gut eine Fläche/Membran zu erkennen.

Anschließend erfolgt die Bestimmung der Ausbeutepunkte, zu den oben definierten Betriebspunkten. Es hierbei unerheblich, ob die Ausbeute für jeden Betriebspunkt in einer Simulation (z.B. Modell Spyro) berechnet oder die Ausbeute im Laborversuch empirisch mittels Versuch für jeden Betriebspunkt erzeugt und gemessen wird. Sofern keine Interpolation stattfindet, ist es sogar unbeachtlich, ob die Betriebsparameter exakt oder ungenau eingestellt werden.

Falls nötig oder gewünscht (um Rechenzeit zu sparen) erfolgt eine Interpolation der berechneten Ausbeutepunkte. Sofern die Berechnung mittels Spyro bzw. Messung im Technikums-/Laborversuch eines Ausbeutepunktes länger dauert als das Interpolieren zwischen zweier Betriebspunkte und ist der Interpolationsfehler in Relation zur gewünschten Genauigkeit klein genug, bzw. sind die Kosten pro Ausbeutepunkt zu hoch, ist es effizient, nur wenige Betriebspunkte zur Simulation bzw. im Versuch zu verwenden und hinterher Zwischenschritte zu interpolieren.

Zuvor wurden die 4 Parameter jeweils 3 mal variiert, dieser Raster wird nun für den kommenden Schritt deutlich verfeinert. Die COT wird zwischen 840 und 860 °C 41 mal variiert (d.h. in 0.5 Grad Schritte), D/KW von 0.4 - 0.8 21mal, FLOWR von 18-22 9 mal und COP von 1.7-1.9 5 mal. Dies ergibt in Summe 41*21*9*5 = 38745 Betriebspunkte.

Ist man an der Konzentration von z.B. 6 Komponenten (H2, CH4, C2H4, C2H6, C3H6, C3H8) interessiert, so werden diese nun mittels PCA abgebildet. Es zeigt sich, dass die relevante Information in den ersten 3 "scores" der PCA zu finden ist, d.h. man muss nur die ersten 3 beachten. Trägt man die Ausbeutepunkte in der "Score"-Darstellung in 3D gegeneinander auf, so zeigt sich eine Punktewolke. Die Einhüllende dieser Punktewolke ist die Reaktionsfläche, bzw. Reaktions-Membran. Die PCA liefert die Transformationskoeffizienten zwischen dem Eingangsraum (6 Komponenten) und den Ausgangsraum (3 Scores). Will man einen beliebigen Ausbeutepunkt in den "Score" Raum abbilden, muss man ihn nur mit den Transformationskoeffizienten multiplizieren. Will man einen beliebigen Punkt aus dem "Score" Raum in den Ausbeuteraum abbilden, muss man dessen Koordination nur durch die Transformationskoeffizienten dividieren.In Fig. 4a-d sind zwei Reaktionsflächen 14, 14' für geringfügig unterschiedliche Ausgangszusammensetzungen eingezeichnet, so dass sich Form (grob anhand der jeweiligen Grenze 19, 19' bzw. deren Projektionen ersichtlich) und Lage (siehe insbesondere den Versatz in x-Richtung gemäß Fig. 4c) der beiden Reaktionsflächen ebenfalls nur geringfügig unterscheidet, d.h. die zweite Reaktionsfläche 14' ist gegenüber der ersten Reaktionsfläche 14 nur wenig rotiert bzw. verschoben.

Zum Vergleich sind in Fig. 5 die Reaktionsflächen 14", 14"' für völlig unterschiedliche Ausgangszusammensetzungen (z.B. Ethan und Naphtha) eingezeichnet. In diesem Fall liegen die Reaktionsflächen 14", 14'" weit auseinander und unterscheiden sich auch in ihrer Form deutlich.

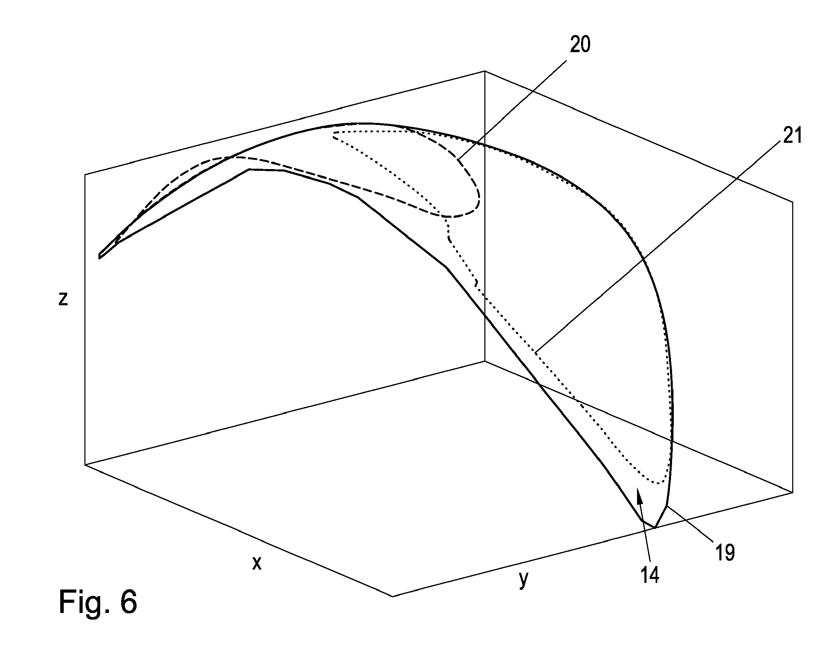

Wie in Fig. 6 ersichtlich, können unterschiedliche Verarbeitungseinheiten 2, z.B. unterschiedliche Reaktortypen oder Öfen, naturgemäß unterschiedliche Betriebsfenster aufweisen, welche unterschiedlichen Grenzen 19, 20, 21 für eine Reaktionsfläche entsprechen. Da die Reaktionsfläche 14 sämtliche Betriebsparameter-Konfigurationen enthält und somit die Form und Lage der Reaktionsfläche von der Variation eines (weiteren) Betriebsparameters unabhängig ist, gehen die durch die Grenzen 19, 20, 21 definierten Flächenabschnitte in eine gemeinsame, zusammenhängende Reaktionsfläche 14 über. Abhängig vom Verlauf der jeweiligen Grenze 19, 20, 21 sind bei einem bestimmten Ausgangsstoff für verschiedene Verarbeitungseinheiten 2 jeweils nur die auf der Reaktionsfläche 14 innerhalb der Grenze 19, 20, 21 liegenden Produktzusammensetzungen erreichbar bzw. herstellbar.

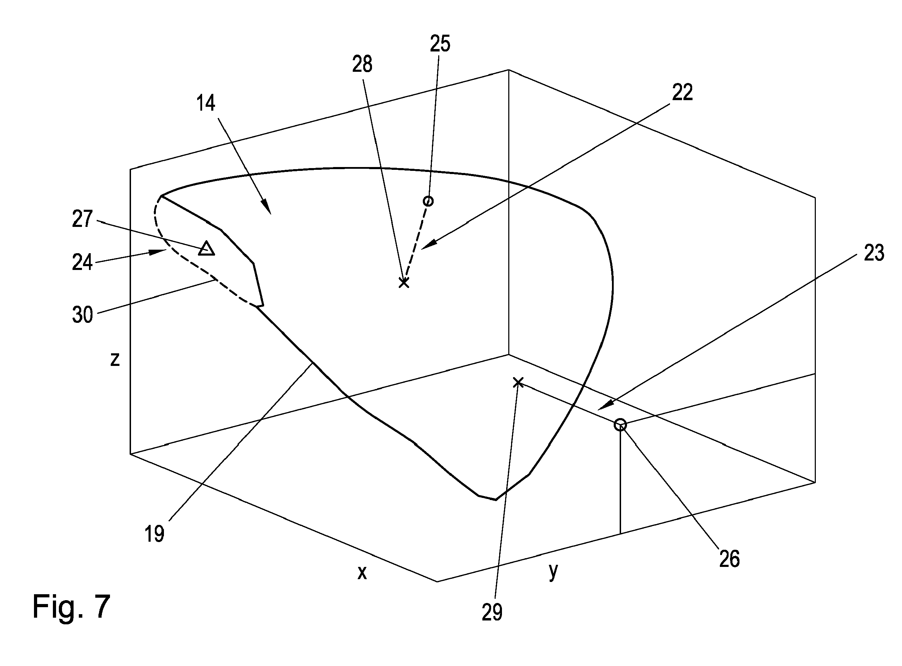

Fig. 7 zeigt schematisch drei Anwendungsfälle 22, 23, 24, bei welchen die Reaktionsfläche 14 mit einer gemessenen Produktzusammensetzung 25, 26, 27 in Beziehung gesetzt wird. Der erste Anwendungsfall 22 betrifft die Erkennung und Korrektur eines Messfehlers bei der Messung der Produktzusammensetzung. Zur Erkennung eines Fehlers wird zunächst überprüft, ob die gemessene Produktzusammensetzung 25 in der Reaktionsfläche 14 liegt. In diesem Fall wäre von der Richtigkeit der Messung auszugehen und die Berechnung eines Korrekturwerts erübrigt sich. Im dargestellten Anwendungsfall 22 liegt die Produktzusammensetzung 25 abseits der Reaktionsfläche 14, so dass ein Fehler erkannt wird. Eine bevorzugte Möglichkeit, um die Abweichung der Produktzusammensetzung 21 von der Reaktionsfläche 14 zu ermitteln ist, den der gemessenen Produktzusammensetzung 25 nächstliegenden Punkt auf der Reaktionsfläche 14 als korrigierte Produktzusammensetzung 28 zu wählen. Die Korrektur ergibt sich demzufolge aus dem Abstand zwischen der gemessenen Produktzusammensetzung 25 und der korrigierten Produktzusammensetzung 28. Aufgrund der Wahl des nächstliegenden Punktes entspricht dieses Verfahren einer Minimierung des notwendigen Korrekturwerts, d.h. anschaulich ist der so ermittelte Korrekturwert der kleinste Korrekturwert, welcher die gemessene Produktzusammensetzung 25 in die Reaktionsfläche 14 verschiebt. Bei der Bewertung des Abstands im Rahmen der Suche nach dem nächstliegenden Punkt werden vorzugsweise sämtliche gemessenen Produktkomponenten berücksichtigt, wobei die Differenzen der einzelnen Anteile der Produktkomponenten besonders bevorzugt mit den jeweils zugeordneten Messfehlern gewichtet werden können. Mit einer solchen Gewichtung wird eine den Messfehlern entsprechende Verteilung der Abweichung auf die einzelnen Produktkomponenten erzielt, d.h. es wird eine verhältnismäßig größere Korrektur bei einer Produktkomponente mit verhältnismäßig größerem zugeordneten Messfehler vorgenommen.

Der zweite Anwendungsfall 23 in Fig. 7 betrifft eine Ergänzung einer teilweise gemessenen Produktzusammensetzung 26. In diesem Fall wurde der Anteil von zwei der drei dargestellten Produktkomponenten y, z gemessen und der Anteil der dritten Produktkomponente x ist unbekannt. In Kenntnis der Ausgangszusammensetzung kann in diesem Fall die anwendbare Reaktionsfläche 14 ermittelt werden und es kann jener Punkt auf der Reaktionsfläche 14 als ergänzte Produktzusammensetzung 29 ermittelt werden, an welchem die beiden gemessenen Komponenten y, z der teilweise gemessenen Produktzusammensetzung 26 entsprechen. Da eine solche Zuordnung nicht immer eindeutig sein muss, können im Fall von mehreren möglichen Produktzusammensetzungen z.B. grobe Kenntnisse der Betriebsparameter berücksichtigt werden um das betrachtete Betriebsfenster, d.h. die Grenze des infrage kommenden Bereichs auf der Reaktionsfläche 14, einzuengen.

Bei dem dritten Anwendungsfall 24 handelt es sich um ein Beispiel für einen Modellfehler bzw. einen Fehler in der Parametrierung des Simulationsmodells. Hierbei liegt die gemessene Produktzusammensetzung 27 zwar grundsätzlich auf der Reaktionsfläche, jedoch außerhalb der durch das betrachtete Betriebsfenster festgelegten Grenze 19. Zur Korrektur eines derartigen Fehlers wird durch entsprechende Anpassungen am Betriebsfenster bzw. am Modell eine neue Grenze 30 ermittelt, welche die gemessene Produktzusammensetzung 27 einschließt.

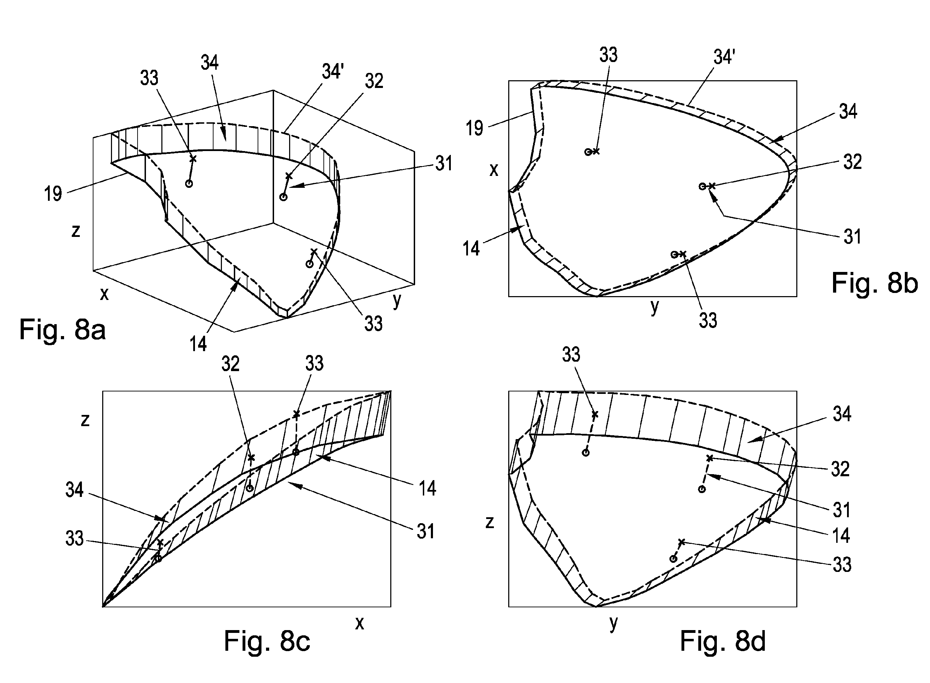

Fig. 8a-d zeigen einen vierten Anwendungsfall 31, bei dem - vergleichbar dem ersten Anwendungsfall 22 - eine außerhalb der Reaktionsfläche 14 mit der Grenze 19 gelegene Produktzusammensetzung gemessen wurde. Falls z.B. wegen mehrfacher unabhängiger Messungen oder wegen gleichbleibendem Ausgangsstoff und gleichbleibender gemessener Produktzusammensetzung 32 von einer korrekten Messung der Produktzusammensetzung ausgegangen werden kann, wird ein Fehler der Messung der Ausgangszusammensetzung angenommen. Zur Behebung des Fehlers wird die kleinste Korrektur der gemessenen Ausgangszusammensetzung gesucht, welche eine Verschiebung der Reaktionsfläche 14 über die gemessene Produktzusammensetzung 32 erzielt. In Fig. 8a-d ist die der korrigierten Ausgangszusammensetzung entsprechende verschobene Reaktionsfläche 34 gestrichelt, d.h. mit gestrichelter Grenze 34', dargestellt. Aufgrund der großen Anzahl an Freiheitsgraden, insbesondere bei Ausgangszusammensetzungen mit drei, vier oder mehr Komponenten, welche sich auf die Form und Lage der Reaktionsfläche auswirken, ist es vorteilhaft, wenn zur Ermittlung der Korrektur mehrere gemessene Produktzusammensetzungen 32, 33 verwendet werden. Durch die Anzahl der gemessenen Punkte kann eine Abstandsfunktion (in der Art eines Volumskeils) zwischen der ursprünglichen Reaktionsfläche 14 und der korrigierten Reaktionsfläche 34 konstruiert werden, welche anschließend der Korrektur der Reaktionsfläche 14 dient.

Grundsätzlich gibt es hierbei mehrere Möglichkeiten welcher Abstand berechnet wird - welche auch kombiniert werden können. Wie der gewählte Abstand rein mathematisch berechnet wird, ist unerheblich, wobei sich die hier gezeigte Berechnung mittels Punktewolke als außerordentlich effizient erwiesen hat. Der gemessene Ausbeutepunkt wird hierzu mittels PCA in den "Score" Raum der Reaktionsfläche abgebildet.

### a) Minimaler Abstand - d.h. keine Berücksichtigung des Betriebspunktes (falls man dem gemessenen Betriebsparameter nicht traut, bzw. diese Information ausblenden will)

In diesem Fall wird der Abstand eines jeden Ausbeutepunktes der Punktewolke der Reaktionsfläche mit dem gemessenen Ausbeutepunkt im "Score" Raum berechnet und der kleinste so berechnete Abstand wird als der minimale gewählt. Da nur endlich viele Ausbeutepunkte in der Punktewolke existieren, ist dies nur eine Näherung, wobei man die Abweichung falls nötig durch Verfeinerung der Interpolation nahezu beliebig verkleinern kann. Sollte eine Kontrolle ergeben, dass die Punktewolke im Bereich des minimalen Abstandes zu "dünn" ist, kann in diesem Bereich mittels z.B. Interpolation die Punktewolke verdichtet und der minimale Abstand erneuert berechnet werden. Dem Ausbeutepunkt in der Punktewolke der Reaktionsfläche, der den minimalen Abstand aufweist, sind natürlich auch die entsprechenden Betriebsparameter zugewiesen. Diese können mit den "tatsächlichen" verglichen und eine Korrektur der Betriebsparameter berechnet werden.

### b) Abstand zwischen der Abbildung des gemessenen Betriebspunktes auf die Reaktionsfläche und der gemessenen Ausbeute

Falls z.B. Ausbeutekorrekturen berechnet werden sollen, die auf den gemessenen Betriebsparametern basieren, oder wissen will, wie eine Einsatzänderung Betriebspunkte (bzw. das operating window) verschiebt, ist der Abstand zwischen der Abbildung des gemessenen Betriebspunktes auf die Reaktionsfläche (d.h. die Ausbeute des Betriebspunktes wird mittels Simulation oder Versuch ermittelt und die Ausbeute mittels PCA auf die Reaktionsfläche abgebildet) und des gemessenen Ausbeutepunktes.

### c) Fixieren einiger Betriebsparameter und Anpassung/Korrektur anderer

Sollen Betriebsparameter korrigiert werden und weiß man, dass einige "exakt" (d.h. nicht korrigiert werden sollen) stimmen und andere nur ungefähr, kann aus der Punktewolke die Teilmenge herausgegriffen bzw. interpoliert werden, bei der z.B. die Einsatzmenge FLOWR exakt 20 t/h beträgt, die COT jedoch zwischen 840 und 860 schwanken darf. Der minimale Abstand wird in Folge von dieser Teilmenge aus zu dem gemessenen Ausbeutepunkt bestimmt und nur die COT korrigiert.

Auch wenn die Interpolation und Berechnung des Abstandes von jedem Punkt der Punktewolke zu jedem gemessenen Punkt sehr aufwendig erscheint, ist dies dank der heutigen Rechenkapazität in deutlich < 1 Sekunde erledigt. Man könnte zur Berechnung des Abstandes z.B. ebenso die Einhüllende der Punktewolke lokal durch Regression annähern und von dieser Näherung den minimalen Abstand bestimmen, dies ist aber weder robuster noch schneller.

Soll der Abstand zwischen 2 Reaktionsflächen ermittelt werden ( z.B. um Einsätze oder Spaltcoils/Reaktoren gesamtheitlich und von Betriebspunkten losgelöst vergleichen zu können), wiederholt man das Prozedere ganz einfach Punkt für Punkt und ermittelt so den Volumskeil.

Zur Identifikation des Fehlers, nachdem ein Fehler erkannt wurde, entsprechend einem der Anwendungsfälle 22, 23, 24, 31 können insbesondere Wechselwirkungen bzw. Vergleiche mehrerer parallel laufender Verarbeitungsprozesse herangezogen werden. Beispielsweise ist es im Fall von systematisch bei mehreren Verarbeitungseinheiten 6 auftretenden Fehler wahrscheinlich, dass entweder die Zusammensetzung eines von den betroffenen Verarbeitungseinheiten 6 gemeinsam verwendeten Ausgangsstoffs fehlerhaft bestimmt wurde oder, insbesondere wenn alle Verarbeitungsprozesse betroffen sind, dass es sich um einen fehlerhaften Modellparameter handelt.

Beispielsweise ist es vorteilhaft, bei drei unterschiedlichen Einsätzen (E1, E2, E3) an der Anlagengrenze, welche für fünf unterschiedliche Öfen (O1 - O5) unterschiedlich gemischt verwendet werden, die Auswertung aller Verarbeitungsprozesse (Öfen) zu kombinieren. In diesem Fall hat jeder Ofen eine andere Einsatzmischung, obwohl es nur drei Ausgangszusammensetzungen der Einsätze gibt. Würde man die Einsatzmessungs- bzw. auch die einsatzbezogene Spyro-Modell-Korrektur jeweils getrennt pro Ofen vornehmen, ergäbe sich z.B. folgendes Szenario: es wird 8 mal pro Stunde und Ofen das Spaltgas (Produkt) gemessen und pro Einsatz sind 5 Parameter zu bestimmen/korrigieren. Dann sind pro Ofen zur Korrektur der Einsatzmischung bzw. des Spyro-Modells 8 Messpunkte/h vorhanden, um 5 Parameter des Einsatzes bzw. Spyro-Modells zu bestimmen. Eine eindeutige Fehlerzuordnung was vom Spyro-Modell kommt und was vom Einsatz ist kaum möglich. Man muss sich daher darauf beschränken z.B. den Einsatz zu korrigieren und das Verhältnis von Messpunkten zu Parametern ist 8/5 bzw. über alle Öfen 8*5/5*5 = 8/5 (weil jeder Ofen separat behandelt wird). Eine Korrekturverbindung zwischen den Öfen besteht in diesem Fall nicht.

Sieht die Zuordnung Einsätze zu Öfen etwa wie folgt aus:

| Ofen | Einsatz E1/E2/E3 in m% |
|---|---|
| O1 | 80/20/0 |
| O2 | 80/0/20 |
| O3 | 50/40/10 |
| O4 | 0/80/20 |
| O5 | 70/10/20 |

kann man die Einsatzkorrektur Ofen und Einsatz übergreifend bestimmen: Der Einsatz E1 wird z.B. bei 01/2/3/5 verwendet. Daher gibt es für E1 4*8=32, für E2 4*8=32 und für E3 4*8=32 Spaltgasmessungen mit denen sich jeweils 5 Parameter der Einsätze E1, E2 und E3 bestimmen lassen, bzw. gibt es in Summe 32*3=96 Messungen für 15 Parameter. Hierzu wird die Korrektur nicht auf die Ofeneinsatzmischung angewendet, sondern auf die Ausgangseinsätze, d.h. die Ausgangszusammensetzungen der ursprünglichen (ungemischten) Einsätze. Gleichzeitig lassen sich Spyro- und Einsatzfehler korrigieren, da die Einsatzkorrektur ofenübergreifend stimmen muss, Spyro aber auch pro Ofen korrigiert werden kann. Durch das wesentlich bessere Verhältnis von Messungen zu Parametern 96/15=6.4 im Vergleich zu 8/5=1.6, ist diese Korrektur wesentlich robuster und genauer und Fehler die von z.B. der Spaltgasmessung pro Ofen kommen, werden im "Least Squares" Sinne ausgemittelt und verfälschen das Ergebnis weniger. Zusätzlich sind die Betriebspunkte ofenübergreifend pro Einsatz viel breiter auf der Reaktionsfläche verteilt, wodurch die Robustheit nochmals stark angehoben wird. Ein Ofen wird in einem Betriebspunkt betrieben, dadurch streuen die Punkte auf der Fläche unter Anwendung eines Ofens wenig, wodurch es schwierig sein kann, eine Fläche eindeutig hineinzulegen. Bei mehreren Öfen gibt es hingegen mehrere Betriebspunkte, die auf der Reaktionsfläche verteilt sind. Dadurch kann man die Fläche besser schätzen.

Falls ein Einsatz definitiv bekannt ist (z.B. reines Ethan), kann dieser Ausgangseinsatz natürlich konstant gehalten werden und bei den Mischungen werden nur die anderen Einsätze angepasst. Dies kann die Einsatzkorrektur auch vereinfachen, wenn man z.B. weiß, dass 10 m% Ethan in der Mischung enthalten sein muss, weil eben Ethan mit 10 m% hinzugemischt wird.

Natürlich können auch und gerade im Fall eines fehlerhaften Modellparameters die Messungen sämtlicher Verarbeitungsprozesse gemeinsam als wesentlich größere Datenbasis zur Ermittlung einer Korrektur des (globalen) Modellparameters verwendet werden, so dass Fehler schneller und genauer bestimmt werden können und der Einfluss von tatsächlich lokalen Messfehlern minimiert wird. Umgekehrt kann bei gleichbleibendem Ausgangsstoff und somit gleichbleibender Ausgangszusammensetzung von einer Veränderung der gemessenen Produktzusammensetzung auf ein Problem bei der Produktanalyse, d.h. bei der Messung der Produktzusammensetzung, geschlossen werden. Wenn allerdings bei angenommenem gleichbleibendem Ausgangsstoff eine konsistente Verschiebung der Reaktionsfläche und der gemessenen Produktzusammensetzung (d.h. kein feststellbarer Fehler) beobachtet wird, deutet dies auf eine tatsächliche Veränderung der Ausgangszusammensetzung und einen Fehler z.B. bei der Bereitstellung (Transport, Mischung, etc.) des Ausgangsstoffs hin.

## Patentansprüche

1. Computergestütztes Verfahren zur Analyse eines chemischen Verarbeitungsprozesses, bei welchem aus einem Ausgangsstoff (1) ein Produkt (3) erzeugt wird, dessen Produktzusammensetzung neben einer Ausgangszusammensetzung des Ausgangsstoffs (1) von zumindest zwei Betriebsparametern des Verarbeitungsprozesses abhängt, durch Simulation des Verarbeitungsprozesses, wobei
- die Ausgangszusammensetzung gemessen oder vorgegeben wird,
- ausgehend von der Ausgangszusammensetzung und unter Variation der zumindest zwei Betriebsparameter eine Reaktionsfläche (14) von erwarteten Produktzusammensetzungen simuliert wird, wobei jeder Punkt auf der Reaktionsfläche (14) zumindest einer Konfiguration der Betriebsparameter entspricht,
- eine Produktzusammensetzung (25; 26; 27; 32, 33) zumindest teilweise gemessen wird, und
- die zumindest teilweise gemessenen Produktzusammensetzung (25; 27; 32, 33) mit der Reaktionsfläche (14) zur Erkennung von Mess- oder Simulationsfehlern, zur Erkennung einer Änderung der Ausgangszusammensetzung oder zur Ergänzung der gemessenen Produktzusammensetzung (26) in Beziehung gesetzt wird.

2. Verfahren nach Anspruch 1, **dadurch gekennzeichnet, dass** die Reaktionsfläche (14) eine höchstens dreidimensionale, vorzugsweise nur zweidimensionale, Menge von Produktzusammensetzungen ist, wobei die Anzahl der Dimensionen der Reaktionsfläche (14) der Anzahl der physikalischen Freiheitsgrade des Produkts (3) entspricht.

3. Verfahren nach Anspruch 1 oder 2, **dadurch gekennzeichnet, dass** eine Abweichung der zumindest teilweise gemessenen Produktzusammensetzung (25; 32, 33) von der Reaktionsfläche (14) ermittelt wird und die ermittelte Abweichung zur Erkennung eines Fehlers bei der Messungen der Ausgangszusammensetzung, bei der Messung der Produktzusammensetzung oder in der Simulation verwendet wird.

4. Verfahren nach Anspruch 3, **dadurch gekennzeichnet, dass** ein Korrekturwert für die Messung der Produktzusammensetzung (25; 32, 33) aus der ermittelten Abweichung ermittelt wird, wobei vorzugsweise aus mehreren gemessenen Produktzusammensetzungen (32, 33) eine Abstandsfunktion zwischen diesen und der Reaktionsfläche (14) ermittelt wird, welche Abstandfunktion einem von der gemessenen Produktzusammensetzung (32, 33) abhängigen Korrekturwert entspricht.

5. Verfahren nach einem der Ansprüche 1 bis 3, **dadurch gekennzeichnet, dass** ein Fehler in der Simulation korrigiert wird, indem zumindest ein Modellparameter eines der Simulation zugrunde liegenden Modells des Verarbeitungsprozesses so verändert wird, dass die gemessene(n) Produktzusammensetzung(en) (32, 33) in einer auf Basis des zumindest einen veränderten Modellparameters aktualisierten Reaktionsfläche (34) liegt bzw. liegen.

6. Verfahren nach Anspruch 5, **dadurch gekennzeichnet, dass** bei einem Verarbeitungsprozess mit mehreren Reaktoren, wobei die gemessene Produktzusammensetzung die Gesamtheit der Produkte der Reaktoren betrifft, ein einem einzelnen Reaktor zugeordneter Modellparameter oder ein mehreren Reaktoren zugeordneter Modellparameter so geändert wird, dass eine lokal möglichst glatte Reaktionsfläche erzielt wird.

7. Verfahren nach Anspruch 1 oder 2, **dadurch gekennzeichnet, dass** ein einer unvollständig gemessenen Produktzusammensetzung (26) nächstliegender Punkt der Reaktionsfläche ermittelt wird und die unvollständig gemessenen Produktzusammensetzung (26) durch die diesem Punkt entsprechende, erwartete Produktzusammensetzung (29) ergänzt wird.

8. Verfahren nach Anspruch 7, **dadurch gekennzeichnet, dass** bei der Ermittlung des nächstliegenden Punkts (28) der Reaktionsfläche eine den einzelnen Komponenten der gemessenen Produktzusammensetzung (25) zugeordnete Messgenauigkeit berücksichtigt wird, indem die Abstände der einzelnen Komponenten mit der Messgenauigkeit gewichtet werden.

9. System zur Analyse eines chemischen Verarbeitungsprozesses mit einer Produktanalyseeinheit (8) zur Messung der Produktzusammensetzung eines Produkts (3) des Verarbeitungsprozesses, wobei die Produktanalyseeinheit (8) mit einer Auswerteeinheit (6) verbunden ist, **dadurch gekennzeichnet, dass** die Auswerteeinheit (6) eingerichtet ist, ausgehend von einer, vorzugsweise mit einer ebenfalls mit der Auswerteeinheit (6) verbundenen Ausgangsanalyseeinheit (5) zur Messung der Ausgangszusammensetzung eines Ausgangsstoffs (1) des Verarbeitungsprozesses, gemessenen oder vorgegebenen Ausgangszusammensetzung und unter Variation von zumindest zwei Betriebsparametern des Verarbeitungsprozesses eine Reaktionsfläche (14) von erwarteten Produktzusammensetzungen zu simulieren und eine von der Produktanalyseeinheit (8) gemessene Produktzusammensetzung mit der Reaktionsfläche (14) in Beziehung zu setzen.

10. System nach Anspruch 9, **dadurch gekennzeichnet, dass** die Auswerteeinheit (6) ein Simulationsmodul (10) zur Simulation der Reaktionsfläche (14) und ein Vergleichsmodul (12), welches zur Erkennung eines Fehlers der Ausgangsanalyseeinheit (5), der Produktanalyseeinheit (8) und/oder des Simulationsmoduls (10) sowie zur Ermittlung einer Korrektur zur Behebung des Fehlers eingerichtet ist, wobei das Korrekturmodul (12) zur Übermittlung der ermittelten Korrektur vorzugsweise mit der Ausgangsanalyseeinheit (5), der Produktanalyseeinheit (8) und/oder dem Simulationsmodul (10) verbunden ist.
